(19) Europäisches Patentamt / European Patent Office / Office européen des brevets

(11) **EP 2 793 774 B1**

(12) **FASCICULE DE BREVET EUROPEEN**

(45) Date de publication et mention de la délivrance du brevet:
**22.03.2017 Bulletin 2017/12**

(21) Numéro de dépôt: **12819107.9**

(22) Date de dépôt: **21.12.2012**

(51) Int Cl.:
*A61F 13/00* (2006.01)     *A61F 15/00* (2006.01)
*A61B 10/00* (2006.01)     *A61M 1/00* (2006.01)

(86) Numéro de dépôt international:
**PCT/FR2012/053068**

(87) Numéro de publication internationale:
**WO 2013/093380 (27.06.2013 Gazette 2013/26)**

(54) **DISPOSITIF NON-INVASIF, DESTINE A RECUEILLIR LES EXSUDATS D'UNE PLAIE, SON UTILISATION ET KIT COMPRENANT LEDIT DISPOSITIF**

NICHTINVASIVE VORRICHTUNG ZUM ENTFERNEN VON EXSUDATEN AUS EINER WUNDE, VERWENDUNG DAVON UND KIT MIT DIESER VORRICHTUNG

METHOD OF MICROSCOPIC AND MACROSCOPIC ANALYSIS OF THE PROGRESSION OF WOUND HEALING

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **22.12.2011 FR 1162295**

(43) Date de publication de la demande:
**29.10.2014 Bulletin 2014/44**

(73) Titulaires:
- **Urgo Recherche Innovation et Développement
  21300 Chenove (FR)**
- **Université Paul Sabatier Toulouse III
  31400 Toulouse (FR)**
- **Institut National de la Santé et de la Recherche Médicale (INSERM)
  75013 Paris (FR)**

(72) Inventeurs:
- **DARDENNE, Christophe
  31470 Fontenilles (FR)**
- **PIPY, Bernard
  31400 Toulouse (FR)**
- **LAMOISE, Michel
  21110 Bessey Les Citeaux (FR)**

(74) Mandataire: **Cabinet Plasseraud
66 rue de la Chaussée d'Antin
75440 Paris Cedex 09 (FR)**

(56) Documents cités:
DE-A1-102008 041 785     US-A- 3 486 504
US-A1- 2005 222 528     US-A1- 2005 222 544
US-A1- 2007 191 754     US-A1- 2010 256 545

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

**Description**

Domaine d'application :

La présente invention concerne un dispositif médical permettant de recueillir facilement les exsudats issus d'une plaie afin de les analyser en temps réel, permettant ainsi un suivi cellulaire, biochimique et moléculaire de l'avancée de la cicatrisation de la plaie, et qui permet également d'observer visuellement l'évolution de la cicatrisation d'une plaie.

Art Antérieur :

La cicatrisation d'une plaie est un processus physiopathologique naturel, les tissus humains et animaux étant capables de réparer des lésions localisées par des processus de réparation et de régénération qui leur sont propres.
La rapidité et la qualité de la cicatrisation d'une plaie dépendent de l'état général de l'organisme atteint, de l'étiologie de la plaie, de l'état et de la localisation de la plaie, et de la survenue ou non d'une infection, ainsi que des facteurs génétiques prédisposant ou non à des troubles de la cicatrisation.
La cicatrisation naturelle d'une plaie se déroule principalement selon trois phases successives, chacune de ces phases étant caractérisée par des activités cellulaires spécifiques qui font progresser le processus de réparation selon des séquences chronologiques précises : la phase inflammatoire, la phase de granulation (ou phase proliférative), et la phase de maturation, donnant l'aspect définitif de la cicatrice.
La première phase, la phase inflammatoire, débute dès la rupture des vaisseaux sanguins qui déclenche la formation d'un caillot (coagulation du sang) principalement composé de fibrine et de fibronectine, et qui va constituer une matrice provisoire. Cette matrice comble en partie la lésion et va permettre la migration au sein de la zone lésée des cellules inflammatoires recrutées pour assurer la détersion de la plaie. Les plaquettes présentes vont également libérer des facteurs (par exemple des cytokines, des facteurs de croissance) permettant le recrutement de cellules impliquées dans le processus cicatriciel. Cette phase se caractérise par l'infiltration et l'activation sur le site de la lésion, de nombreuses cellules inflammatoires (tels les polynucléaires neutrophiles), mais aussi des cellules assurant le nettoyage de la plaie ou détersion (tels les macrophages).
La seconde phase correspond au développement du tissu de granulation. On observe d'abord une colonisation de la blessure par prolifération des fibroblastes. Puis, la migration des cellules endothéliales à partir des vaisseaux sains va permettre la néovascularisation, ou angiogenèse, du tissu lésé. Dans le tissu de granulation, les fibroblastes sont activés et vont se différencier en myofibroblastes présentant des propriétés contractiles importantes, générées par les microfilaments d'actine, permettant la contraction de la plaie. Ces microfilaments sont exprimés par une protéine : l'actine $\alpha$-musculaire lisse. Ces myofibroblastes jouent donc un rôle majeur dans la formation et la maturation du tissu de granulation qui va conduire à la cicatrisation de la lésion. Il y a ensuite migration des kératinocytes et reconstruction de l'épiderme.
Cette phase est initiée par une diminution de l'état inflammatoire de la lésion s'accompagnant d'une apoptose des cellules inflammatoires.
La troisième phase du processus est une étape principalement de maturation visant à reconstruire un tissu fonctionnel le plus identique possible au tissu d'origine. Le tissu de granulation précédemment formé subit donc un remodelage. Une partie de la matrice extracellulaire est digérée par des protéases (essentiellement des métallo-protéases matricielles et des élastases), et on observe une réorganisation progressive de la matrice extracellulaire. Progressivement, le collagène de type III, majoritaire dans le tissu de granulation, est remplacé par le collagène de type I, principal composant matriciel du derme. A la fin de la phase de maturation, les fibroblastes, myofibroblastes et cellules vasculaires voient leur prolifération et/ou leur activité réduites. Puis, les cellules excédentaires meurent par apoptose. Parallèlement au remodelage de la matrice extracellulaire et à l'apoptose des cellules excédentaires.
La phase inflammatoire est une phase essentielle de la cicatrisation et doit être transitoire. Dans certains cas, elle peut être prolongée par la présence de certains agents infectieux comme *Staphylococcus aureus* ou *Pseudomonas aeruginosa* ou par une pathologie préexistante comme le diabète, une déficience immunitaire ou encore une insuffisance veineuse. La résolution de l'inflammation est un point critique qui conditionne l'amorçage de la réparation tissulaire. Une perturbation de cette phase va engendrer un prolongement anormal de la phase inflammatoire et aboutir à la chronicité de la lésion en retardant la réparation tissulaire. La résolution de l'inflammation est un phénomène dynamique qui implique l'apoptose des cellules inflammatoires, leur élimination et l'apparition de médiateurs anti-inflammatoires, chimiotactiques et angiogéniques. Une manière d'évaluer le bon déroulement de cette période critique, qu'est la phase de résolution, est d'analyser les exsudats provenant de la plaie (cellules et médiateurs). En effet, la chronicité inflammatoire se traduit par un afflux plus important de cellules inflammatoires, une production excessive de nombreuses cytokines et chimiokines inflammatoires et de protéases qui dégradent la matrice cellulaire. Inversement, la phase de résolution implique l'apparition d'un certain type de macrophages responsables de la phagocytose des cellules inflammatoires apoptotiques. Ces macrophages sont aussi impliqués dans la production de médiateurs favorisant la phase de granulation ainsi que la réparation tissulaire. L'analyse de l'exsudat permettra au médecin d'évaluer la phase inflammatoire et sa résolution, et permettra ainsi de choisir de traiter la plaie par un agent thérapeutique adapté de manière à favoriser une meilleure cicatrisation.

Traditionnellement, les plaies sont recouvertes par un pansement ou une compresse pour empêcher une contamination par des agents infectieux présents dans l'environnement, mais également pour maintenir la plaie dans un milieu humide favorisant la cicatrisation. Cependant, ce système de protection ne permet pas d'analyser les exsudats et de visualiser la cicatrisation en temps réel. De plus, l'exsudat s'accumule dans le pansement et sature le milieu absorbant du pansement. Le pansement doit donc être changé régulièrement pour éviter la macération de la plaie, ce qui implique un risque de contamination de la plaie qui est exposée momentanément à l'air libre. De plus, les pansements peuvent également adhérer, même faiblement à la plaie, et induire ainsi des lésions à la plaie lors de leur retrait. Ces nouvelles agressions engendrent une poursuite de l'inflammation, qui a pour conséquence de prolonger la phase inflammatoire et donc de modifier de façon substantielle la cinétique de cicatrisation, ce à quoi le dispositif de la demanderesse remédie de façon efficace.

Des chambres implantables ont été mises au point pour étudier la cicatrisation de plaies du derme sur des animaux tels que la souris ou le porc. Cependant, ces chambres implantables sont insérées en partie sous la peau de l'animal et sont maintenues en place par des points de suture. Cette méthode est donc invasive, c'est à dire qu'elle présente comme inconvénient majeur de nécessiter un acte chirurgical et risque donc de provoquer une réaction inflammatoire chez le sujet en question, en créant des agressions tissulaires lors de son implantation. Ces agressions tissulaires auront des conséquences néfastes sur le bon déroulement de la cicatrisation. De plus, ces chambres implantables ont essentiellement été mises au point pour modéliser les mécanismes de cicatrisation chez l'animal.

Enfin, la société ACUEITY, dans son brevet WO 2004/045674, décrit un dispositif pour recueillir l'exsudat provenant des glandes mammaires. Le dispositif en question est constitué d'un réservoir contenant un tampon absorbant qui est en contact avec le mamelon et le dispositif est maintenu en place au-dessus du mamelon par un adhésif. L'exsudat est absorbé par le tampon et ne peut donc pas être analysé en temps réel ; de plus les exsudats récoltés au sein du tampon ne sont pas purs et des risques de perte et/ou de dégradation au moment de leur extraction du tampon peuvent être constatés. Ce dispositif n'est pas muni d'un système d'ouverture et doit donc être enlevé afin de récupérer les exsudats du tampon à des fins d'analyse.

La demande de brevet US 2007/0191754 décrit un dispositif préformé de protection de blessure comprenant un cadre constitué d'une ou plusieurs couches d'un matériau et un revêtement approprié. Le matériau absorbant ou transportant les exsudats peut également constituer l'une de deux ou de plusieurs couches de matériau formant le cadre.

Il existe donc un réel besoin en un dispositif non-invasif, étanche et conformable, permettant de recueillir directement et passivement des exsudats purs à des fins d'analyse en temps réel (notamment afin de contrôler le déroulement de phase inflammatoire et le démarrage de la phase de granulation) et permettant de visualiser la progression de la cicatrisation sans être nécessairement obligé d'ouvrir ledit dispositif, ni de le retirer, assurant ainsi la protection de l'environnement intérieur du dispositif, et donc la protection de la plaie, vis à vis de l'environnement extérieur (c'est-à-dire vis-à-vis des agents infectieux extérieurs, mais aussi de l'animal lui-même, ou de ses congénères, autrement dit, vis-à-vis du grattage, des morsures, des salissures, des chocs, ou encore des frottements) et vis à vis du dispositif lui-même.

Invention :

L'invention est telle que définie dans les revendications.

Un premier objet de la présente invention est un dispositif non-invasif, permettant de recueillir les exsudats d'une plaie, comprenant une chambre ayant une paroi latérale, une extrémité inférieure ouverte et une extrémité supérieure, une ouverture d'accès étant disposée sur l'extrémité supérieure et/ou sur la paroi latérale de ladite chambre, ledit dispositif étant destiné à être en contact avec la peau et à circonscrire la plaie lors de son utilisation par l'intermédiaire d'une interface.

Ledit dispositif comprend un moyen de fermeture amovible de la chambre, destiné à être disposé en position fermée, sur l'ouverture d'accès de la chambre.

Lors de son utilisation, le dispositif selon l'invention est simplement collé sur la peau saine de façon à circonscrire la plaie du patient sans entrer en contact avec ladite plaie grâce à son interface adhésive. Lors de la cicatrisation, la plaie suinte et l'exsudat s'écoule dans le dispositif. Une fois qu'une quantité d'exsudat suffisante pour les analyses a été recueillie dans le dispositif, l'exsudat est prélevé, par exemple en ouvrant le moyen de fermeture. Il est à noter de plus, que lorsque l'exsudat est prélevé régulièrement, la plaie ne macère pas ce qui favorise une cicatrisation rapide.

Le dispositif selon l'invention peut notamment être considéré comme un dispositif passif de récolte des exsudats, en opposition à un dispositif actif. Par dispositif actif, on entend un dispositif où les exsudats sont récoltés grâce à l'application d'un effet ou d'une contrainte externe, physique et/ou mécanique, telle qu'une pression négative, comme par exemple les dispositifs sous vide ou les dispositifs de thérapie par pression négative, également appelée TPN. Les dispositifs actifs et passifs peuvent également se différencier par la qualité des exsudats récoltés. En effet, en instaurant une contrainte mécanique et/ou physique dans le dispositif actif, les exsudats récoltés sont d'un volume plus important, et on assiste ainsi à une dilution des différentes cellules, médiateurs, ou facteurs cellulaires contenus dans lesdits exsudats. De plus, avec les dispositifs actifs, les cellules retrouvées dans les exsudats peuvent être mortes et/ou activées. Par cellules activées on entend des cellules extraites de leur milieu naturel se retrouvant en situation de stress et qui peuvent alors perdre, entre autres, leurs capacités fonctionnelles, ce qui est, par exemple, le cas pour des macrophages et des polynucléaires neutrophiles. L'avantage du dispositif de la présente invention est donc de permettre de conserver l'intégrité et les fonctionnalités des différentes populations cellulaires contenues dans les exsudats issus de la plaie. On obtient ainsi une vraie « image » de la plaie.

De plus, le dispositif de la présente invention est un dispositif de récolte directe des exsudats sur le site cicatriciel. Par « dispositif de récolte directe », on entend un dispositif dans lequel les exsudats sont directement prélevés à leur source d'émission, dans le cas présent au voisinage de la plaie, l'exsudat n'ayant donc pas besoin de transiter par une chambre ou réservoir connexe et/ou par un matériau absorbant avant son extraction du dispositif. La récolte directe permet ainsi de conserver l'intégrité et la physiologie de la plaie et de son environnement, le tout sans perturber la cicatrisation de la plaie. Ainsi, le dispositif selon l'invention est centré sur l'analyse macroscopique et microscopique des exsudats issus de la plaie, mais aussi de la plaie elle-même.

Ledit dispositif selon l'invention est dépourvu de tout moyen absorbant entrant en contact avec l'exsudat et la plaie. Cela permet d'éviter une macération de la plaie due à la saturation dudit moyen absorbant par l'exsudat comme cela peut être le cas avec un pansement. De plus, l'absence de moyen absorbant permet d'éviter tout piégeage ou dégradation des protéines, métabolites et cellules présentes dans l'exsudat, tout ceci dans l'objectif de récupérer des exsudats purs et sans perte de cellules et de métabolites.

Selon un mode de réalisation particulier, le dispositif est étanche au niveau de la zone d'interaction dispositif/peau du sujet. Ainsi, l'exsudat recueilli dans la chambre ne peut pas s'échapper par les bords dudit dispositif. Il est également possible de rajouter une lèvre en matériau semi rigide conformable, éventuellement à mémoire de forme, tel que notamment le PMMA (polyméthacrylate de méthyle), le POM-C (copolymère de polyacétal), le PEKK (polyéther kétone kétone), le silicone, le polyuréthane ou encore le polyamide, sur la partie inférieure de l'élément de maintien, lèvre qui peut se révéler utile lorsque la surface sur laquelle le dispositif est fixé n'est pas plane, permettant ainsi d'assurer encore d'avantage, l'étanchéité dudit dispositif. Selon un mode de réalisation particulier, le dispositif comprend un moyen de réaliser des échanges gazeux entre l'intérieur dudit dispositif et l'air extérieur afin d'assurer une bonne cicatrisation de la plaie en évitant l'hypoxie et/ou l'anoxie. Ledit moyen de réaliser des échanges gazeux est préférentiellement un filtre qui permet de maintenir l'intérieur du dispositif stérile, c'est-à-dire d'éviter une contamination par l'environnement extérieur. Des exemples de filtres utilisables sur le dispositif selon l'invention peuvent notamment être des filtres réalisés en polyuréthane, des non tissés, des frittés (disques poreux de silice compressée) ou encore une membrane stérile. Le filtre peut notamment être placé sur, sous ou dans le moyen de fermeture, mais aussi sur, sous ou dans les parois latérales de la chambre, la partie externe dudit filtre étant toujours en contact avec l'environnement extérieur au dispositif.

Selon un mode de réalisation particulier, le dispositif comprend un système de graduation pour évaluer la quantité d'exsudat contenue dans le dispositif.

La chambre du dispositif peut notamment être un élément de forme tubulaire, préférentiellement réalisée en matériau rigide tel que le PMMA (polyméthacrylate de méthyle), le POM-C (copolymère de polyacétal), le PEKK (polyéther kétone kétone) ou le polyamide. Eventuellement, la chambre du dispositif pourra être réalisée en matériau souple tel que notamment le polyuréthane ou le silicone.

La chambre de forme tubulaire doit avoir un volume interne suffisamment élevé pour recueillir tout l'exsudat généré par une plaie soit 5000 $g/m^2/24h$. Cependant, afin de ne pas trop déranger le patient, la hauteur du dispositif devrait être préférentiellement inférieure à 1 centimètre. Afin de mieux caractériser cette chambre, il est possible de la décrire par sa contenance maximale en exsudats qui est inférieure à 1mL/$cm^2$, préférentiellement inférieure à 700$\mu$L/$cm^2$, et de façon encore plus préférentielle, inférieure à 500$\mu$L/$cm^2$.

Le dispositif selon l'invention comprend, en outre, un élément de maintien, éventuellement de forme tubulaire, circonscrit ou inscrit à ladite chambre, ouvert à ses deux extrémités. Par « élément de maintien circonscrit à la chambre », on entend, au sens de la présente invention, un élément de maintien qui entoure par l'extérieur la paroi latérale de la chambre dans sa partie inférieure. Par « élément de maintien inscrit dans la chambre », on entend, au sens de la présente invention, un élément de maintien qui est disposé à l'intérieur de la chambre, à proximité de la paroi latérale et dans la partie inférieure de la chambre.

Selon un mode de réalisation préféré, l'élément de maintien de forme tubulaire est réalisé en matériau semi-rigide à mémoire de forme, tel que notamment le PMMA (polyméthacrylate de méthyle), le POM-C (copolymère de polyacétal), le PEKK (polyéther kétone kétone), le silicone, le polyuréthane ou le polyamide.

Selon un autre mode de réalisation préféré, l'élément de maintien et la chambre tubulaire sont dotés chacun d'un moyen permettant de les solidariser. Ce moyen peut être un pas de vis, un clip, une goupille, un système de baïonnette, un système de verrouillage et/ou éventuellement de la colle.

Lorsque le dispositif présente un élément de maintien circonscrit à la chambre, la section de la chambre tubulaire est inférieure à la section de l'élément de maintien. Lorsque le dispositif présente un élément de maintien inscrit dans la chambre, la section de la chambre tubulaire est supérieure à la section de l'élément de maintien. Dans l'un ou l'autre de ces modes de réalisation, il importe que la plus petite ouverture de l'extrémité inférieure du dispositif ait une surface supérieure à celle de la plaie à traiter, de façon à la circonscrire. Dans le cas où l'élément de maintien est inscrit dans la chambre tubulaire, la section de ladite chambre peut se rétrécir au niveau de sa partie supérieure, tel un goulot de bouteille. Dans un autre mode de réalisation, la partie supérieure de ladite chambre pourra avoir un diamètre supérieur à l'élément de maintien, ou bien le même diamètre, auquel cas, la visualisation de la plaie sera complète.

De nombreuses formes peuvent être envisagées, aussi bien pour la section de la chambre tubulaire que pour celle de l'élément de maintien, telles que notamment circulaire, ovale, elliptique, carrée, rectangulaire. Selon un mode de réalisation préféré, la chambre et l'élément de maintien ont la même forme. Cependant, il n'est pas nécessaire que la chambre et l'élément de maintien aient la même forme. On peut, par exemple, envisager d'adapter une chambre tubulaire cylindrique sur un élément de maintien parallélépipédique.

Selon un mode de réalisation préféré, l'extrémité inférieure de la chambre tubulaire et/ou de l'élément de maintien peut notamment présenter une collerette conformable externe réalisée en matériau souple, tel que par exemple du polyuréthane ou du silicone.

Selon un autre mode de réalisation particulier, ladite collerette peut être un élément dissocié de la chambre ou de l'élément de maintien. Elle se caractérise en un élément perforé en matériau souple, matériau souple choisi parmi la liste des matériaux souples tels que définis précédemment, et vient se circonscrire autour de la partie externe de l'ensemble élément de maintien/chambre tubulaire, et ce de façon la plus proximale possible de cet ensemble.

Le dispositif selon l'invention est destiné à être en contact avec la peau et à circonscrire la plaie lors de son utilisation par l'intermédiaire d'une interface qui est un adhésif disposé sur une jupe en matériau souple. Ladite jupe en matériau souple est maintenue entre la paroi latérale de la chambre et l'élément de maintien avec un moyen permettant de solidariser ces trois éléments, tels qu'un pas de vis, un clip, une goupille, un système de verrouillage et/ou éventuellement de la colle. Le matériau souple de la jupe peut notamment être choisi parmi le groupe comprenant des supports souples conformables tels que des films, des mousses, des silicones et des non tissés. Cette souplesse permet d'adapter le dispositif sur des zones non-planes telles que notamment une phalange, un genou ou un coude. Ainsi par jupe, on entend au sens de la présente invention, un élément comprenant une seule perforation, pouvant notamment être évasé et avoir une forme de tronc de cône. L'interface peut notamment être plane lorsqu'elle n'est pas intégrée au dispositif et prendre une forme évasée lorsqu'elle est fixée entre la chambre tubulaire et l'élément de maintien.

L'étanchéité dudit dispositif est préférentiellement assurée par la juxtaposition de la chambre tubulaire et de l'élément de maintien, constituants entre lesquels on retrouve intercalée la jupe en matériau souple. Cette dernière conférant en plus à l'ensemble du dispositif des propriétés de conformabilité de bonne facture. Par conformabilité de bonne facture on entend que la jupe en matériau souple s'adapte sur la surface à laquelle elle est fixée, en prenant une forme adéquate. Le dispositif, une fois fixé sur une surface plane ou une surface non plane, permet de conserver la totalité du volume des exsudats récoltés au niveau de la plaie. Il en est de même pour le volume d'une solution qui est injectée dans ledit dispositif au travers d'un septum retrouvé sur la chambre ou sur le moyen de fermeture. L'étanchéité dudit dispositif est calculée par l'intermédiaire d'une méthode de mesure relativement simple et aisée de mise en oeuvre décrite à l'exemple 4.

Cette méthode est mise en oeuvre aussi bien sur une surface plane que sur une surface non plane. La mise en oeuvre de cette méthode sur une surface non plane permet de mesurer l'étanchéité dudit dispositif objet de l'invention dans des situations où la conformabilité de ce dernier apparaît primordiale.

Il est à noter que quelque soit le type de surface, par exemple une surface plane ou non plane, sur laquelle le dispositif est fixé, l'étanchéité dudit dispositif est démontrée. Ainsi, le dispositif objet de la présente invention allie étanchéité et conformabilité au moyen de la juxtaposition de la chambre tubulaire, de l'élément de maintien et de la jupe en matériau souple.

L'interface ne doit pas entrer en contact avec la plaie du patient. Elle est fixée au niveau du pourtour des berges de la plaie sur la peau saine du patient, le tout dans un but de circonscription de la plaie. L'interface doit être suffisamment résistante pour que le dispositif puisse tenir plusieurs jours sur la peau sans que l'étanchéité, au niveau de la zone peau/dispositif, du dispositif n'en soit affectée.

La jupe peut avoir différents types de formes possibles, telles que des formes simples, carrées, rondes, rectangulaires, triangulaires, à bords arrondis, ou plus élaborées en forme d'étoiles, ou encore en forme de trèfle, dont les branches peuvent être ou non de même dimensions, afin de pouvoir s'adapter à l'endroit du corps sur lequel le dispositif est fixé. Pour la forme trèfle, celle-ci présente l'avantage de ne pas autoriser de plissures au niveau de ses parties proximales de la chambre, et permet également une conformabilité améliorée selon la position occupée sur la partie du corps du sujet sur laquelle est positionné le dispositif.

Selon un mode de réalisation préféré, la surface libre de l'interface est recouverte d'une barrière protectrice telle que notamment un film plastique. Cette barrière protectrice préserve les propriétés adhésives du dispositif et empêche une contamination éventuelle par des poussières ou par des germes avant l'utilisation dudit dispositif.

On peut également prévoir que l'adhésif constituant ladite interface soit pré-enduit c'est-à-dire que l'adhésif ait été appliqué lors de la fabrication du dispositif ou alors enduit par l'utilisateur au moment de l'utilisation du dispositif, c'est-à-dire une fois que le dispositif est sorti de son emballage de protection. L'enduction peut notamment être effectuée au moyen d'un pinceau, ou encore d'une spatule, c'est-à-dire par enduction manuelle, avant de positionner ledit dispositif sur la peau du sujet.

L'adhésif, constituant l'interface peut notamment être choisi dans le groupe comprenant des colles chirurgicales, des filmogels, de la nitrocellulose, du collodion, du cyanoacrylate, des masses hydrocolloïdes, des adhésifs acryliques, des adhésifs UV ou encore des adhésifs « Hot Melt ».

Le moyen de fermeture amovible dudit dispositif permet d'accéder facilement à la plaie. Ledit moyen de fermeture amovible s'adapte sur la (ou les) ouverture(s) d'accès à la chambre, préférentiellement sur l'extrémité supérieure de la chambre, par un pas de vis, un clip, une goupille, un moyen de sertissage et/ou un système de baïonnette. De préférence, ledit moyen de fermeture adapté sur la chambre en position fermée, par l'intermédiaire par exemple d'un pas de vis, permet de conserver le milieu clos. La jonction entre ledit moyen de fermeture et la chambre, assurée par tout moyen cité *supra* comme par exemple par l'intermédiaire d'un pas de vis, est donc étanche.

Ce moyen de fermeture peut préférentiellement être réalisé en matériau semi-rigide ou rigide tel que le PMMA (polyméthacrylate de méthyle), le POM-C (copolymère de polyacétal), le PEKK (polyéther kétone kétone), le polyamide, le silicone, ou encore par toute matière métallique, telle que l'aluminium.

Selon un mode de réalisation particulier, ledit moyen de fermeture amovible est relié à la chambre tubulaire par un moyen de liaison, tel que notamment un lien plastique. Cela permet d'éviter de perdre ledit moyen de fermeture lorsque celui-ci est séparé de la chambre tubulaire. Selon un mode de réalisation particulier le moyen de fermeture peut être facilement retiré afin de photographier ou filmer la plaie et son évolution.

Selon un mode de réalisation préféré, le dispositif comprend un moyen de visualisation de la plaie. Ainsi la chambre tubulaire et le moyen de fermeture peuvent être réalisés en matériaux transparents. On peut également prévoir une fenêtre transparente, munie éventuellement d'une lentille grossissante, sur le moyen de fermeture du dispositif. L'avantage de ce moyen de visualisation est de pouvoir suivre l'évolution de la cicatrisation ou de toute infection qui pourrait survenir. De plus, cette surveillance est effectuée sans intervenir directement sur la plaie ce qui permet d'éviter toute mauvaise manipulation de la plaie ou sa réouverture. De surcroît, cela permet de visualiser la plaie sans que celle-ci ne soit exposée à l'environnement extérieur qui peut contenir des agents pathogènes, gage de non-contamination de l'intérieur de la chambre tubulaire. Le moyen de fermeture peut également être muni d'un diaphragme. Lorsque le diaphragme est en position ouverte, l'utilisateur peut voir la plaie et lorsque le diaphragme est en position fermée, la plaie n'est plus visible.

Il est important de noter que le dispositif objet de la présente invention est un dispositif dont l'utilité finale n'est pas de traiter à proprement parler la plaie. Il s'agit de préférence d'un moyen permettant le suivi aussi bien macroscopique que microscopique de la plaie, de l'exsudat et de son environnement cellulaire et moléculaire comme on le retrouve décrit au travers de la demande de brevet déposée par les Laboratoires Urgo sous le numéro FR 11 62344.

Selon un mode de réalisation préféré, le dispositif comprend au moins un septum ou une microvanne destiné à prélever ou injecter du liquide. Ce septum permet notamment de prélever l'exsudat sans enlever le dispositif ce qui préserve la stérilité du milieu interne. Ce septum peut également être utilisé pour injecter une solution afin de nettoyer ou désinfecter la plaie. Le septum peut également être utilisé pour injecter une solution contenant un agent pharmacologique ou un agent thérapeutique tel que notamment un antibiotique, un antidouleur, un anti-infectieux, ou encore un agent cicatrisant. De par l'absence d'un moyen absorbant dans le dispositif, la solution injectée sera en contact direct avec la plaie, contrairement à un pansement. Le septum peut être placé sur le moyen de fermeture ou sur la paroi de la chambre tubulaire et éventuellement de l'élément de maintien. Avantageusement, deux canalisations comprenant des moyens de fermeture, tels que des septums ou des microvannes, peuvent être positionnés de part et d'autre de la paroi latérale de la chambre tubulaire afin de faciliter le lavage de la plaie. Ainsi, la solution nettoyante, par exemple, est injectée par un septum, circule dans le dispositif, et est extraite par le deuxième septum.

L'exsudat peut également être collecté dans le dispositif, à des fins d'analyse, par ouverture du moyen de fermeture ou directement par prélèvement « in situ ». L'exsudat recueilli contient tous les facteurs et métabolites produits lors de la cicatrisation. L'exsudat contient également des cellules vivantes et fonctionnelles qui peuvent éventuellement être remises en culture afin de réaliser des études phénotypiques et fonctionnelles, ex vivo. De surcroît, l'exsudat peut contenir des agents pathogènes, tels que des bactéries, qui peuvent être facilement identifiés après prélèvement ce qui permettra au médecin de prescrire un traitement adapté.

On peut également combiner un ou plusieurs modes de réalisation du dispositif selon l'invention. Ainsi, par exemple, l'invention peut porter sur un dispositif qui comprend au moins un septum ou une microvanne, destiné à prélever ou injecter du liquide et/ou un moyen apte à réaliser des échanges gazeux entre l'intérieur dudit dispositif et l'extérieur.

L'invention sera mieux comprise à la lumière des figures suivantes qui ne sont qu'illustratives et ne sauraient en rien limiter l'invention.

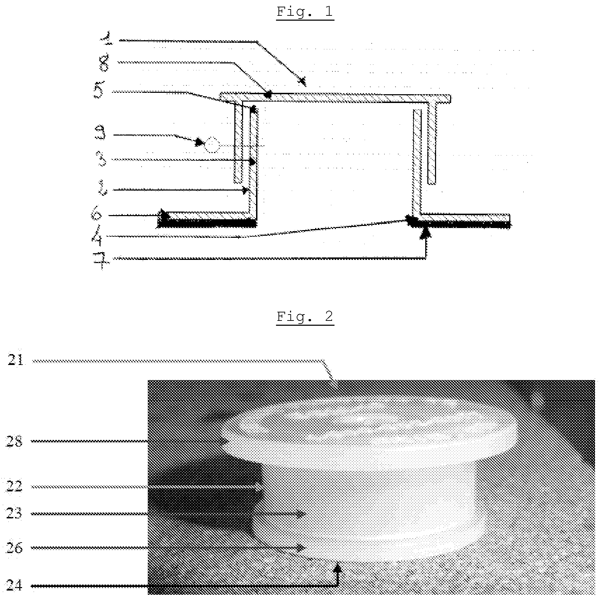

La figure 1 est une vue en coupe transversale d'un dispositif qui n'est pas selon l'invention. Le dispositif (1) est constitué d'une chambre tubulaire (2) comprenant une paroi latérale (3), et deux extrémités, inférieure (4) et supérieure (5), ouvertes. Ladite extrémité inférieure (4) de la chambre tubulaire (2) présente une collerette (6) externe en matériau souple. L'interface (7) du dispositif (1) est constituée d'une couche mince de colle chirurgicale, appliquée sur la surface inférieure de la collerette (6) de la chambre tubulaire (2). Un moyen de fermeture (8) du dispositif (1) est fixé à la partie supérieure de la chambre tubulaire (2) au moyen d'une goupille (9).

La figure 2 représente une photographie d'un dispositif (21) qui n'est pas selon l'invention comprenant une chambre tubulaire (22) ayant une paroi latérale (23) et deux extrémités, inférieure (24) et supérieure, ouvertes. L'extrémité inférieure (24) présente une collerette (26) externe. Un moyen de fermeture (28) est fixé à la partie supérieure de la chambre tubulaire (22) au moyen d'un clip (non représenté ici).

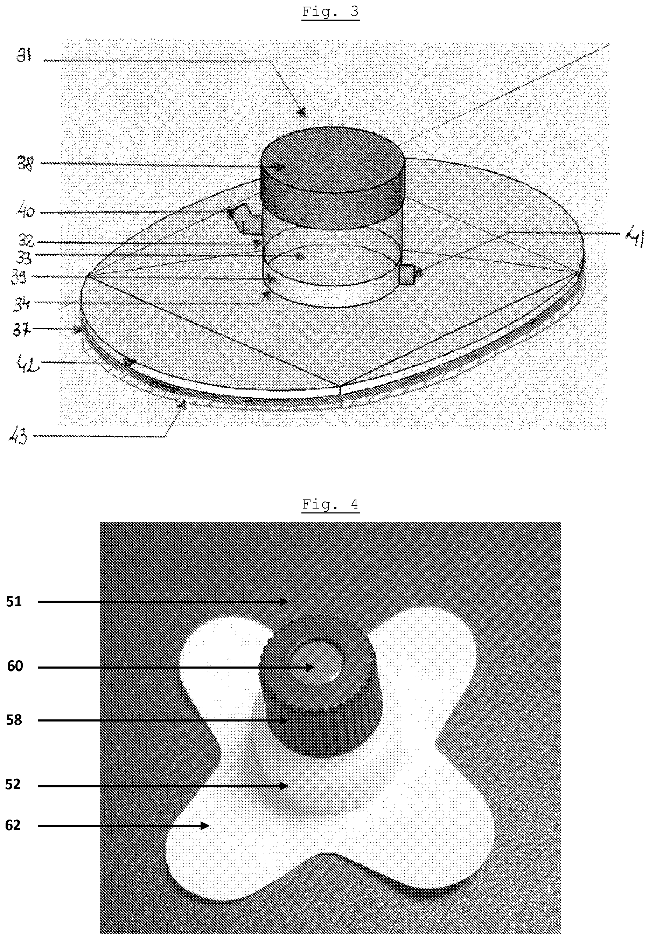

La figure 3 est une représentation schématique éclatée d'un dispositif (31) selon l'invention constitué d'une chambre tubulaire (32) de forme cylindrique et d'un élément de maintien (39) ayant une section de diamètre inférieur au diamètre de la chambre tubulaire (32). Ledit élément de maintien (39) est donc inscrit dans l'extrémité inférieure (34) de ladite chambre tubulaire (32) et maintenu par un clip. La paroi latérale (33) de la chambre tubulaire (32) est munie de deux septums (40 et 41)) diamétralement opposés, qui permettent de réaliser un lavage facile de la plaie. Le deuxième septum (41) étant situé au niveau de l'élément de maintien (39), ce dernier est donc muni d'une ouverture ayant sensiblement la même taille que celle du septum (41). L'interface (37), est constituée d'un adhésif disposé sur la surface inférieure d'une jupe (42) en matériau souple qui a pour forme un élément comprenant une seule perforation, évasé, en forme de tronc de cône. Ladite jupe est fixée entre l'élément de maintien (39) et la paroi latérale (33) de la chambre tubulaire (32) ce qui permet de maintenir la jupe en place. La surface libre de l'interface (37) adhésive est protégée par un film protecteur (43). Un moyen de fermeture (38) est fixé à la partie supérieure de la chambre tubulaire (32) par un pas de vis (non visible ici).

La figure 4 représente une photographie d'un autre mode de réalisation dudit dispositif selon l'invention. Ce dispositif (51) se compose d'une chambre tubulaire (52), d'un élément de maintien inscrit dans la chambre tubulaire (52) et fixé au moyen d'un clip (non visible ici). L'interface est constituée d'un adhésif disposé sur la surface inférieure d'une jupe (62) en matériau souple qui est maintenue en place entre l'élément de maintien et la paroi latérale de la chambre tubulaire (52). La surface libre de l'interface est protégée par un film protecteur (non visible ici). Un moyen de fermeture (58) s'adapte sur la partie supérieure de la chambre tubulaire (52) par un pas de vis. Ledit moyen de fermeture (58) est muni en son centre d'un septum (60) qui est utilisé pour prélever l'exsudat à l'intérieur du dispositif (51) sans avoir à ouvrir le dispositif.

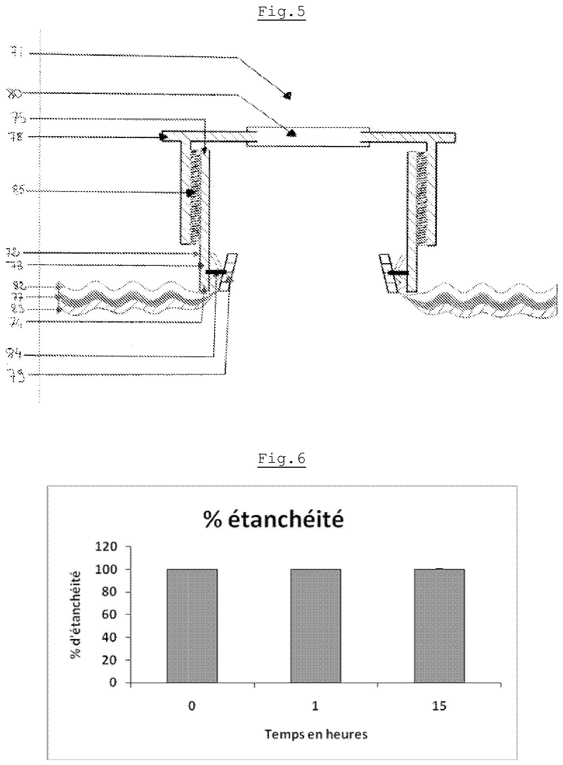

La figure 5 est une vue en coupe transversale d'un dispositif (71) selon l'invention, constitué d'une chambre tubulaire (72) comprenant une paroi latérale (73), et deux extrémités, inférieure (74) et supérieure (75), ouvertes. Un élément de maintien (79) est inscrit dans l'extrémité inférieure (74) de ladite chambre tubulaire (72) et est maintenu en place par un clip (84). L'interface (77) du dispositif est constituée d'un adhésif disposé sur la surface inférieure d'une jupe (82) en matériau souple correspondant à un élément tubulaire en forme de tronc de cône, c'est-à-dire que son ouverture supérieure présente un diamètre plus petit que son ouverture inférieure. Ladite ouverture supérieure de la jupe est fixée entre l'élément de maintien (39) et la paroi latérale (33) de la chambre tubulaire (32) par un clip (84) ce qui permet de maintenir la jupe en place. La surface inférieure libre de l'interface (77) adhésive est protégée par un film protecteur (83). Un moyen de fermeture (78) du dispositif (71) est fixé à la chambre tubulaire au moyen d'un pas de vis (85). Ledit moyen de fermeture est réalisé en matériau transparent afin de visualiser la plaie et il est muni en son centre d'un septum (80) afin de recueillir l'exsudat.

La figure 6 est une courbe représentant le pourcentage d'étanchéité du dispositif réalisé selon l'exemple 1, ledit dispositif étant fixé à une surface plane, en fonction du temps.

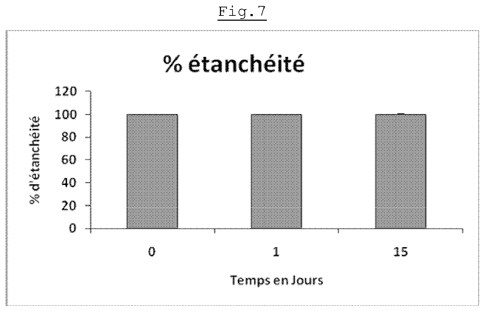

La figure 7 est une courbe représentant le pourcentage d'étanchéité du dispositif réalisé selon l'exemple 1, ledit dispositif étant fixé à une surface non plane, en fonction du temps.

Un autre objet de l'invention est l'utilisation du dispositif décrit précédemment pour suivre et/ou surveiller l'évolution de la cicatrisation d'une plaie. En effet, le dispositif permet de recueillir et d'analyser l'exsudat issu d'une plaie. Pour suivre l'évolution de la cicatrisation d'une plaie, il convient de positionner le dispositif au-dessus de la plaie. Il faut alors attendre qu'une quantité suffisante d'exsudat s'accumule dans le dispositif. Afin d'optimiser la récupération des facteurs et médiateurs produits ainsi que des cellules pouvant adhérer à la plaie, un lavage de la plaie à l'aide d'une solution physiologique peut être envisagé. On peut alors prélever l'exsudat contenu dans le dispositif par ouverture du dispositif ou à l'aide d'une seringue au travers du septum. L'exsudat ainsi prélevé peut être analysé pour connaître l'état d'avancement de la phase inflammatoire et de la cicatrisation en général ou savoir si un agent infectieux est présent dans la plaie.

Selon un mode d'utilisation particulier dudit dispositif, une solution est introduite dans le dispositif après son application sur la peau. Cette solution peut contenir un agent désinfectant et/ou nettoyant qui permet de rendre la plaie et le milieu intérieur du dispositif, propres et stériles. Cette solution peut également contenir un agent thérapeutique ou un agent pharmacologique, tel que notamment un antibiotique, un antidouleur, un anti-infectieux ou encore un agent cicatrisant.

Selon un mode d'utilisation particulier dudit dispositif, les exsudats prélevés dans ce dispositif pourront permettre leur analyse en temps réel.

Selon un mode d'utilisation particulier dudit dispositif, l'exsudat est prélevé à travers un septum ou une microvanne, présent sur le dispositif. Ce prélèvement permet de recueillir l'exsudat sans enlever le dispositif et sans intervenir directement sur la plaie.

Enfin, selon un autre mode d'utilisation particulier dudit dispositif, celui-ci pourra permettre de laver la plaie.

Un autre objet de l'invention concerne une trousse ou kit pour recueillir l'exsudat des plaies comprenant :

- au moins un dispositif tel que décrit précédemment ;
- au moins un dispositif de récupération, tel qu'une micro-pompe, ou une seringue ;
- optionnellement une aiguille.

Selon un mode de réalisation préféré, le kit comprend au moins une solution à injecter dans ledit dispositif. La solution à injecter comprend un principe actif désinfectant et/ou nettoyant et/ou un agent thérapeutique. Cette solution permet de stériliser l'environnement de la plaie et/ou traiter une infection et/ou la plaie.

L'invention va être décrite plus en détails à l'aide des exemples suivants qui sont donnés à titre purement illustratif et non limitatif.

Exemples:

**Exemple 1 : Dispositif pour recueillir l'exsudat issu d'une plaie**

Le dispositif est tel que celui de la figure 5, et comprend :

- une chambre (72) de section circulaire de 0,5 cm de hauteur et 1 cm de diamètre en polyméthacrylate de méthyle ;
- un élément de maintien (79) à section circulaire de 0,2 cm de hauteur et 0,9 cm de diamètre en polyéther cétone cétone qui est inscrit dans la chambre (79) ;
- une interface (77) constituée d'un adhésif acrylique pré-enduit sur la surface inférieure d'une jupe (82) en film souple ;
- un clip (84) permettant de fixer l'élément de maintien (79) et la jupe (82) en film souple à la chambre (72) tubulaire
- un film protecteur (83) en plastique protégeant la surface libre de l'interface adhésive;
- un moyen de fermeture (78) à section circulaire en polyuréthane transparent, afin de pouvoir visualiser la plaie, qui est fixé à la chambre (72) tubulaire par un pas de vis (85) et qui comprend un septum (80) destiné à prélever l'exsudat.

**Exemple 2 : Utilisation du dispositif sur une plaie de 0,75 cm$^2$**

On souhaite suivre la cicatrisation d'une plaie d'environ 0,75 cm$^2$ présente sur l'avant-bras gauche d'un patient.

Pour cela, on utilise le dispositif de l'exemple 1.

On ouvre le système de fermeture (78) du dispositif afin de visualiser la partie basse de la chambre (72) tubulaire et de l'élément de maintien (79). On enlève le film protecteur (83) de l'interface (77) adhésive. On positionne l'interface (77) adhésive sur l'avant-bras gauche du patient autour de sa plaie, en prenant garde à ce que l'interface (77), la chambre (72) tubulaire et l'élément de maintien (79) n'entrent pas en contact avec la plaie. On exerce une légère pression pendant 2 à 3 minutes pour assurer un bon maintien sur l'avant-bras. On visse le moyen de fermeture (78) sur la chambre. A un temps déterminé, on injecte une solution de lavage par le septum (80) du moyen de fermeture (78) de la chambre (72) tubulaire puis on prélève l'exsudat par le septum grâce à une seringue munie d'une aiguille. On analyse l'exsudat afin de vérifier qu'aucun agent infectieux n'est présent dans la plaie.

**Exemple 3 : Kit**

Le kit contient le dispositif selon l'exemple 1 ainsi que deux seringues, deux aiguilles, une solution de lavage et un flacon stérile. Le dispositif stérile est mis en place comme dans l'exemple 2 et stérilisé grâce à la solution de lavage qui est injectée à travers le septum par une seringue munie d'une aiguille selon l'exemple 2. La deuxième seringue et la deuxième aiguille permettent d'évacuer la solution désinfectante après le lavage de la plaie. L'exsudat prélevé est transféré dans le flacon stérile en vue d'analyses biologiques.

**Exemple 4 : Mesure de l'étanchéité du dispositif de l'exemple 1 fixé sur une surface plane ou non plane**

Afin de mesurer l'étanchéité du dispositif réalisé selon l'exemple 1, on fixe celui-ci sur une surface en verre plane ou non plane. Dans le cas présent la surface en verre non-plane est la surface latérale d'une fiole jaugée de 250 mL.

On ajoute alors un volume de 500 μL d'une solution aqueuse, telle que de l'eau, à l'intérieur dudit dispositif. On referme ce dernier grâce au moyen de fermeture. Dès la fermeture du dispositif (temps 0h également appelé par la suite T0), on pèse l'ensemble : support en verre, dispositif fermé, eau.

Cet ensemble est de nouveau pesé 1 heure après (temps 1h également appelé par la suite T1) et 15 heures après (temps 15h également appelé par la suite T15) la fermeture du dispositif.

Le test est réalisé trois fois pour la surface plane et trois fois pour la surface non-plane (numéro 1, 2 et 3).

Afin de déterminer le pourcentage d'étanchéité (appelé par la suite %) à un temps précis, on réalise le calcul suivant :

```
% étanchéité = (poids de l'ensemble au temps T) / (poids initial de
                        l'ensemble à T0).
```

La moyenne du poids sur les trois essais est calculée à T0, T1 et T15 de la manière suivante :

```
Moy(poids) = [poids essai 1 + poids essai 2 + poids essai 3] / 3.
```

La moyenne du pourcentage d'étanchéité sur les trois essais est calculée à T1 et T15 de la manière suivante :

```
Moy(%) = [% essai 1 + % essai 2 + % essai 3] / 3.
```

Les résultats obtenus pour la surface plane sont présentés dans le tableau suivant et l'évolution du pourcentage d'étanchéité en fonction du temps est représentée à la figure 6 :

| SURFACE PLANE 1h | | | |
|---|---|---|---|
| | Poids 0h | Poids 1h | % |
| Numéro 1 | 67,17 | 67,17 | 100 |
| Numéro 2 | 61,29 | | 99,9836841 |
| Numéro 3 | 60,57 | 60,57 | 100 |
| | | | |
| Moy | 63,01 | 63,0066667 | 99,9945614 |
| SEM | | | 0,00543863 |
| | | | |
| SURFACE PLANE 15h | | | |
| | Poids 0h | Poids 15h | % |
| Numéro 1 | 67,17 | 67,163 | 99,9895787 |
| Numéro 2 | 61,29 | 60,978 | 99,4909447 |
| Numéro 3 | 60,57 | 60,56 | 99,9834902 |
| | | | |
| Moy | 63,01 | 62,9003333 | 99,8213378 |
| SEM | | | 0,16520593 |

Le dispositif réalisé selon l'exemple 1 est donc étanche sur surface plane. En effet, l'écart entre les pourcentages d'étanchéité à T+1 heure et T+15 heures n'est pas significatif.

Les résultats obtenus pour la surface non plane sont présentés dans le tableau suivant et l'évolution du pourcentage d'étanchéité en fonction du temps est représentée à la figure 7 :

| SURFACE PLANE 1h | | | |
|---|---|---|---|
| | Poids 0h | Poids 1h | % |
| Numéro 1 | 101,8 | 101,8 | 100 |
| Numéro 2 | 103,41 | 103,38 | 99,9709893 |
| Numéro 3 | 99,74 | 99,74 | 100 |
| | | | |
| Moy | 101,65 | 101,64 | 99,9903298 |
| SEM | | | 0,00967024 |
| | | | |
| SURFACE Non PLANE 15h | | | |
| | Poids 0h | Poids 15h | % |
| Numéro 1 | 101,8 | 101,62 | 99,8231827 |
| Numéro 2 | 103,41 | 103,36 | 99,9516488 |
| Numéro 3 | 99,74 | 99,74 | 100 |
| | | | |
| Moy | 101,65 | 101,57 | 99,9249438 |
| SEM | | | 0,5276032 |

Le dispositif réalisé selon l'exemple 1 est donc étanche sur surface non plane en plus d'être conformable. En effet, l'écart entre les pourcentages d'étanchéité à T+1 heure et T+15 heures n'est pas significatif.

Le dispositif réalisé selon l'exemple 1 allie donc étanchéité et conformabilité grâce à la juxtaposition de la chambre tubulaire, de l'élément de maintien et de la jupe en matériau souple.

**Revendications**

1. Dispositif (31, 51, 71) non invasif permettant de recueillir les exsudats d'une plaie comprenant une chambre (32, 52, 72) ayant une paroi latérale (33, 73), une extrémité inférieure (34, 74) ouverte et une extrémité supérieure (75), une ouverture d'accès étant disposée sur l'extrémité supérieure (75) et/ou sur la paroi latérale (33, 73) de ladite chambre (32, 52, 72), ledit dispositif comprenant en outre un moyen de fermeture (38, 58, 78) amovible de la chambre (32, 52, 72), destiné à être disposé en position fermée sur l'ouverture d'accès de la chambre (32, 52, 72) et un élément de maintien (39, 79), éventuellement de forme tubulaire, circonscrit ou inscrit à ladite chambre (32, 52, 72), ouvert à ses deux extrémités, ledit dispositif (31, 51, 71) étant destiné à être en contact avec la peau et à circonscrire la plaie lors de son utilisation par l'intermédiaire d'une interface (37, 77), **caractérisé en ce que** ladite interface est un adhésif disposé sur une jupe (42, 62, 82) en matériau souple, ladite jupe (42, 62, 82) étant maintenue entre la paroi latérale (33, 73) de la chambre (32, 52, 72) et l'élément de maintien (39, 79).

2. Dispositif (31, 51, 71) selon la revendication 1, **caractérisé en ce que** ledit matériau souple de la jupe (42, 62, 82) est choisi dans le groupe comprenant des supports souples conformables tels que des films, des mousses, des silicones et des non tissés.

3. Dispositif (31, 51, 71) selon la revendication 1 ou 2, **caractérisé en ce que** l'adhésif constituant ladite interface (37, 77) est pré-enduit ou enduit au moment de l'utilisation.

4. Dispositif (31, 51, 71) selon l'une quelconque des revendications précédentes, **caractérisé en ce que** l'élément de maintien (39, 79) et la chambre (32, 52, 72) tubulaire sont dotés chacun d'un moyen permettant de les solidariser.

5. Dispositif (31, 51, 71) selon l'une quelconque des revendications précédentes, **caractérisé en ce que** l'extrémité inférieure (34, 74) de la chambre (32, 52, 72) et/ou de l'élément de maintien (39, 79) comprend une collerette (6, 26) conformable en matériau souple.

6. Dispositif (31, 51, 71) selon l'une quelconque des revendications précédentes, **caractérisé en ce que** ledit élément de maintien (39, 79) est en matériau semi-rigide à mémoire de forme.

7. Dispositif (31, 51, 71) selon l'une quelconque des revendications précédentes, **caractérisé en ce que** ledit moyen de fermeture (38, 58, 78) amovible s'adapte sur la (ou les) ouverture(s) d'accès de la chambre (32, 52, 72) par un pas de vis (85), un clip, une goupille (9), un moyen de sertissage et/ou un système de baïonnette.

8. Dispositif (31, 51, 71) selon l'une quelconque des revendications précédentes, **caractérisé en ce qu'**il comprend au moins un septum (40, 41, 60, 80), ou une microvanne, destiné à prélever ou injecter du liquide et/ou un moyen apte à réaliser des échanges gazeux entre l'intérieur dudit dispositif (31, 51, 71) et l'extérieur.

9. Dispositif (31, 51, 71) selon l'une quelconque des revendications précédentes, **caractérisé en ce qu'**il comprend un moyen de visualisation de la plaie.

10. Utilisation du dispositif (31, 51, 71) tel que défini à l'une quelconque des revendications 1 à 9, pour surveiller la cicatrisation d'une plaie et/ou pour analyser les exsudats de la plaie en temps réel.

11. Kit pour recueillir l'exsudat des plaies comprenant :

   - au moins un dispositif (31, 51, 71) tel que défini à l'une quelconque des revendications 1 à 9 ;
   - au moins un dispositif de récupération, tel qu'une micro-pompe ou une seringue ;
   - optionnellement une aiguille.

**Patentansprüche**

1. Nicht-invasive Vorrichtung (31, 51, 71), welche es erlaubt, die Exsudate einer Wunde zu sammeln, umfassend eine Kammer (32, 52, 72), welche eine laterale Wand (33, 73), ein offenes unteres Ende (34, 74) und ein oberes Ende (75) aufweist, wobei eine Zugangsöffnung an dem oberen Ende (75) oder/und an der lateralen Wand (33, 73) der Kammer (32, 52, 72) angeordnet ist, wobei die Vorrichtung ferner ein von der Kammer (32, 52, 72) lösbares Schließmittel (38, 58, 78) umfasst, welches dazu vorgesehen ist, in geschlossener Position auf der Zugangsöffnung der Kammer (32, 52, 72) angeordnet zu sein, sowie ein an seinen beiden Enden offenes Halteelement (39, 79), gegebenenfalls in rohrförmiger Form, welches die Kammer (32, 52, 72) umschließt oder in ihr eingesetzt ist, wobei die Vorrichtung (31, 51, 71) dazu vorgesehen ist, bei ihrer Verwendung mittels einer Schnittstelle (37, 77) mit der Haut in Kontakt zu sein und die Wunde zu umschließen, **dadurch gekennzeichnet, dass** die Schnittstelle ein Klebstoff ist, welcher auf einem Kragen (42, 62, 82) aus einem elastischen Material angeordnet ist, wobei der Kragen (42, 62, 82) zwischen der lateralen Wand (33, 73) der Kammer (32, 52, 72) und dem Halteelement (39, 79) gehalten ist.

2. Vorrichtung (31, 51, 71) nach Anspruch 1, **dadurch gekennzeichnet, dass** das elastische Material des Kragens (42, 62, 82) aus der Gruppe ausgewählt ist, welche verformbare flexible Träger umfasst, wie beispielsweise Filme, Schäume, Silikone und Vliese.

3. Vorrichtung (31, 51, 71) nach Anspruch 1 oder 2, **dadurch gekennzeichnet, dass** der die Schnittstelle (37, 77) bildende Klebstoff zuvor aufgetragen ist, oder zum Zeitpunkt der Verwendung aufgetragen wird.

4. Vorrichtung (31, 51, 71) nach einem der vorhergehenden Ansprüche, **dadurch gekennzeichnet, dass** das Halteelement (39, 79) und die rohrförmige Kammer (32, 52, 72) jeweils mit einem Mittel versehen sind, welches es erlaubt, sie zu verbinden.

5. Vorrichtung (31, 51, 71) nach einem der vorhergehenden Ansprüche, **dadurch gekennzeichnet, dass** das untere Ende (34, 74) der Kammer (32, 52, 72) oder/und das Halteelement (39, 79) einen anpassbaren Kranz (6, 26) aus flexiblem Material umfasst.

**6.** Vorrichtung (31, 51, 71) nach einem der vorhergehenden Ansprüche, **dadurch gekennzeichnet, dass** das Halteelement (39, 79) aus einem halb-steifen Material mit Formgedächtnis besteht.

**7.** Vorrichtung (31, 51, 71) nach einem der vorhergehenden Ansprüche, **dadurch gekennzeichnet, dass** die lösbaren Schließmittel (38, 58, 78) sich auf die Zugangsöffnung(en) der Kammer (32, 52, 72) durch ein Gewinde (85), einen Clip, einen Arretierstift (9), ein Press-Verbindungsmittel und/oder ein Bajonettsystem einpassen.

**8.** Vorrichtung (31, 51, 71) nach einem der vorhergehenden Ansprüche, **dadurch gekennzeichnet, dass** sie wenigstens ein Septum (40, 41, 60, 80) oder ein Mikroventil umfasst, welches dazu vorgesehen ist, Flüssigkeit zu entnehmen oder einzugeben und/oder ein Mittel, welches dazu eingerichtet ist, Gasaustausch zwischen dem Inneren der Vorrichtung (31, 51, 71) und dem äußeren durchzuführen.

**9.** Vorrichtung (31, 51, 71) nach einem der vorhergehenden Ansprüche, **dadurch gekennzeichnet, dass** sie ein Mittel zum Visualisieren der Wunde umfasst.

**10.** Verwendung der Vorrichtung (31, 51, 71) nach einem der Ansprüche 1 bis 9 zum Überwachen des Verheilens einer Wunde und/oder zum Analysieren der Exsudate der Wunde in Echtzeit.

**11.** Baugruppe zum Sammeln des Exsudates von Wunden, umfassend:

- wenigstens eine Vorrichtung (31, 51, 71) nach einem der Ansprüche 1 bis 9;
- wenigstens eine Rückgewinnungs-Vorrichtung, wie beispielsweise eine Mikropumpe oder eine Spritze;
- optional eine Nadel.

**Claims**

**1.** A non-invasive device (31, 51, 71) which makes it possible to collect the exudates from a wound, comprising a chamber (32, 52, 72) having a side wall (33, 73), an open lower end (34, 74) and an upper end (75), there being an access opening on the upper end (75) and/or on the side wall (33, 73) of said chamber (32, 52, 72), said device further comprising a removable means (38, 58, 78) for closing the chamber (32, 52, 72), intended to be placed in the closed position on the access opening of the chamber (32, 52, 72) and a holding element (39, 79), optionally of tubular shape, which is circumscribed to or inscribed in said chamber (32, 52, 72), and open at both its ends, said device (31, 51, 71) being intended to be in contact with the skin and to surround the wound during use by means of an interface (37, 77), **characterized in that** said interface is an adhesive placed on a skirt (42, 62, 82) made of flexible material, said skirt (42, 62, 82) being held between the side wall (33, 73) of the chamber (32, 52, 72) and the holding element (39, 79).

**2.** The device (31, 51, 71) according to claim 1, **characterized in that** said flexible material of the skirt (42, 62, 82) is chosen from the group comprising conformable flexible supports such as films, foams, silicones and nonwovens.

**3.** The device (31, 51, 71) according to claim 1 or 2, **characterized in that** the adhesive constituting said interface (37, 77) is precoated or coated at the time of use.

**4.** The device (31, 51, 71) according to any one of claims 1 to 3, **characterized in that** the holding element (39, 79) and the tubular chamber (32, 52, 72) each have a means for making them interconnected.

**5.** The device (31, 51, 71) according to any one of the preceding claims, **characterized in that** the lower end (34, 74) of the chamber (32, 52, 72) and/or of the holding element (39, 79) comprises a conformable flange (6, 26) made of flexible material.

**6.** The device (31, 51, 71) according to any one of the preceding claims, **characterized in that** said holding element (39, 79) is made of shape-memory semi-rigid material.

**7.** The device (31, 51, 71) according to any one of the preceding claims, **characterized in that** said removable closing means (38, 58, 78) fits to the access opening(s) of the chamber (32, 52, 72) by means of a screw thread (85), a clip, a pin (9), a crimping means and/or a bayonet system.

8. The device (31, 51, 71) according to any one of the preceding claims, **characterized in that** it comprises at least one septum (40, 41, 60, 80), or one microvalve, intended for sampling or injecting liquid, and/or a means capable of carrying out gas exchanges between the interior of said device (31, 51, 71) and the exterior.

9. The device (31, 51, 71) according to any one of the preceding claims, **characterized in that** it comprises a means for visualizing the wound.

10. The use of the device (31, 51, 71) as defined in any one of claims 1 to 9, for monitoring the healing of a wound, and/or for introducing a solution into the device and/or for analyzing the exudates from the wound in real time.

11. A kit for collecting the exudate from wounds, comprising:

   - at least one device (31, 51, 71) as defined in any one of claims 1 to 9;
   - at least one recovering device, such as a micropump or a syringe;
   - optionally a needle.

Fig. 1

Fig. 2

Fig. 3

Fig. 4

Fig.5

Fig.6

**% étanchéité**

% d'étanchéité

120

100

80

60

40

20

0

0          1          15

Temps en heures

EP 2 793 774 B1

Fig.7

% étanchéité

17

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2004045674 A
- US 20070191754 A

- FR 1162344